# EUROPEAN PATENT APPLICATION

(11) **EP 1 639 942 A1**
(43) Date of publication of application: **29.03.2006**
(21) Application number: 04745920.1
(22) Date of filing: 09.06.2004
(51) Int. Cl.: A61B 5/15, A61B 5/145, G01N 21/27

(54) **DEVICE AND METHOD OF MEASURING LIVING-BODY INFORMATION**

(30) Priority: 12.06.2003 JP 2003168408
(71) Applicant: MATSUSHITA ELECTRIC INDUSTRIAL CO., LTD., Kadoma-shi, Osaka 571-8501 (JP)
(72) Inventor: NAKAYAMA, Hiroshi, Osaka 573-1117 (JP)
(74) Representative: Grünecker, Kinkeldey, Stockmair & Schwanhäusser Anwaltssozietät
(86) International application number: PCT/JP2004/008368
(87) International publication number: WO 2004/110275

(57) **Abstract**

In conventional portable optical blood sugar meters, a lancet device and a sensor device are carried in a state of being separate from each other or in a state of being combined without being changed in size and, therefore, a small amount of blood does not spread over the entire area of a dropwise-application portion when the blood is applied dropwise to the application portion. When an optical measurement is made in such a state, optical information even on a portion where no change in coloration has occurred is also measured, resulting in generation of measurement noise.

A casing 8, a light source 6 which emits light, a photodetector 10 which detects light and a lancet drive mechanism 1, 2, 3, or 4 which drives a detachably attached lancet needle 5 are provided. The lancet needle 5 moves in and out of an opening 9 provided at the extreme tip of the casing 8. Light emitted from the light source 6 is emitted from the opening 9, and light entering the opening 9 reaches the detector 10.

## Description

### Technical Field

The present invention relates to a living body information measuring device, a needle and a living body information measuring method for optically measuring living body information such as a blood sugar value.

### Background Art

In measuring blood sugar value, blood sugar value measurements have been usually made by using a lancet device and a measuring device which are separate from each other and have been used independently of each other. In the case of an optical blood sugar meter, the concentration of glucose contained in blood has been measured in such a manner that blood is applied dropwise to a reaction portion to cause it to react with a reaction reagent through a blood corpuscle filter and a change in coloration thereby caused is measured.

That is, in a case where the blood sugar value is measured with an optical blood sugar meter, blood is first sampled by using a lancet device. Usually, a spring for driving a lancet needle is tensed to enable the lancet device to operate. After tensing the spring of the lancet needle, the lancet device is moved to a part of the body such as an earlobe or the inner surface of a finger from which a blood sample is to be obtained. A trigger button provided in the lancet device is then pressed to release the spring, thereby causing the lancet needle to puncture the part of the body from which a blood sample is to be obtained.

Blood caused by puncture with the lancet needle to bleed is applied dropwise to a reaction portion of an optical blood sugar sensor mounted in the optical blood sugar meter. The blood applied dropwise to the reaction portion of the optical blood sugar sensor reacts with a reaction reagent to cause a change in coloration. The optical blood sugar meter is then made to operate to measure the change in coloration on the reaction portion of the optical blood sugar sensor. The concentration of glucose contained in blood is thus measured.

Thus, the lancet device and the measuring device (optical blood sugar meter) in the conventional optical blood sugar meter have been separate devices.

A device also exists in which a blood sugar sensor measuring instrument and a lancet device are combined into one (see Published Japanese Translation of PCT International Application No. 2001-524680).

There has been a portability problem with the conventional art since the lancet device and the sensor device are carried in a state of being separate from each other or in a state of' being combined without being changed in size.

On the other hand, in a device for measuring living body information, e.g. , an optical blood sugar meter, when a small amount of blood is applied dropwise to the application portion it does not spread over the entire area of a dropwise-application portion. When an optical measurement is made in such a state, even optical information on a portion where no change in coloration has occurred is also measured, resulting in generation of measurement noise.

### Disclosure of the Invention

In view of the above-described problems, an object of the present invention is to provide a living body information measuring device having improved portability and a living body information measuring method.

In view of the above-described problems, another object of the present invention is to provide a living body information measuring device, a needle and a living body information measuring method enabling accurate measurement even when a small amount blood is applied dropwise.

In order to solve the above described problems, the first aspect of the present invention is a living body information measuring device comprising:
a casing;
a light source which is accommodated in said casing and which emits light;
a photodetector which is accommodated in said casing and which detects light; and
a lancet drive mechanism which is accommodated in said casing and which drives a lancet needle,
wherein said lancet needle moves in and out an opening provided in said casing;
the light emitted from said light source is emitted from said opening; and
light entering said opening reaches said detector.

The second aspect of the present invention is the living body information measuring device according to the first aspect of the present invention, wherein the light emitted from said opening is reflected and absorbed by a reagent portion of a sensor; and
the light entering said opening is light reflected by the reagent portion of said sensor.

The third aspect of the present invention is the living body information measuring device according to the first aspect of the present invention, wherein an inner portion of the lancet needle is capable of transmitting light;
the light emitted from said light source passes through the inner portion of said lancet needle and is emitted from said opening; and
the light entering said opening reaches said photodetector by passing through the inner portion of said lancet needle.

The fourth aspect of the present invention is the living body information measuring device according to the third aspect of the present invention, wherein an optical fiber which guides light is inserted in the inner portion of said lancet needle.

The fifth aspect of the present invention is the living body information measuring device according to the first aspect of the present invention, wherein the light emitted from said light source is guided to said opening by an optical fiber and is emitted from said opening; and
the light entering said opening is guided to the photodetector by the optical fiber.

The sixth aspect of the present invention is the living body information measuring device according to the first aspect of the present invention, comprising a computation unit which is accommodated in said casing and which figures out living body information from a result detected by said detector; and
a display which is accommodated in said casing and which displays the figured-out living body information.

The seventh aspect of the present invention is a living body information measuring method using a living body information measuring device having:
a casing;
a light source which is accommodated in said casing and which emits light;
a photodetector which is accommodated in said casing and which detects light; and
a lancet drive mechanism which is accommodated in said casing and which drives a lancet needle, said method comprising:
   a step of causing said lancet needle to move in and out an opening provided in said casing;
   a step of causing the light emitted from said light source to be emitted from said opening; and
   a step of causing light entering said opening to reach said detector.

The eighth aspect of the present invention is a needle used for measurement of living body information, the needle comprising:
a needle main body having a hollow inner portion or a groove provided therein, and
an optical fiber which is inserted in the inner portion or the groove of the needle, and which guides light.

### Brief Description of the Drawings

Figure 1 is a diagram showing the construction of a living body information measuring device in a first embodiment of the present invention;
Figure 2 (a) is a cross-sectional view of the living body information measuring device before puncture in the first embodiment of the present invention;
Figure 2 (b) is a cross-sectional view of the living body information measuring device after puncture in the first embodiment of the present invention;
Figure 3 is a diagram showing the construction of a living body information measuring device in a second embodiment of the present invention;
Figure 4(a) is a cross-sectional view of a living body information measuring device before puncture in a third embodiment of the present invention; and
Figure 4 (b) is a cross-sectional view of the living body information measuring device after puncture in the third embodiment of the present invention.

### (Description of Symbols)

- 1: Spring compressing button
- 2: Spring
- 3: Puncturing needle holder
- 4: Trigger button
- 5: Puncturing needle
- 5a: Puncturing needle
- 6: Light source
- 7: Battery
- 8: Lancet main body
- 9: Opening
- 10: Detector
- 11: Computation unit
- 12: Display
- 14: Optical fiber
- 15: Optical fiber

### Best Mode for Carrying Out the Invention

Embodiments of the present invention will be described with reference to the drawings.

### (First Embodiment)

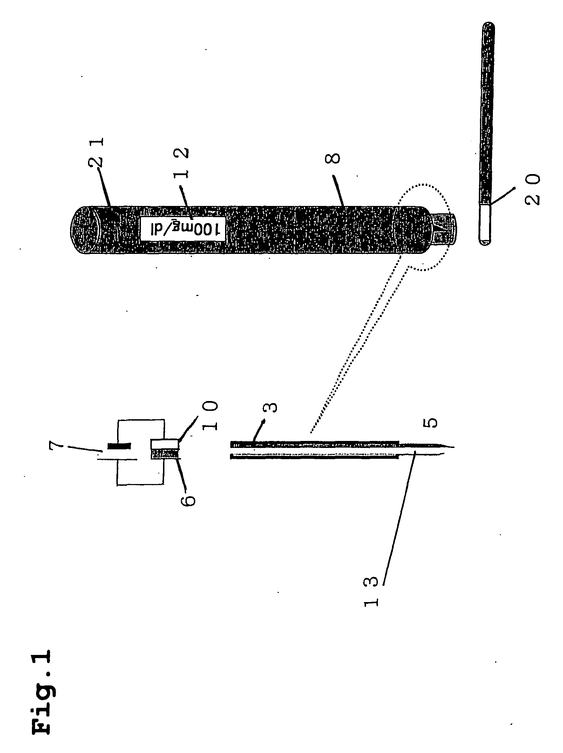

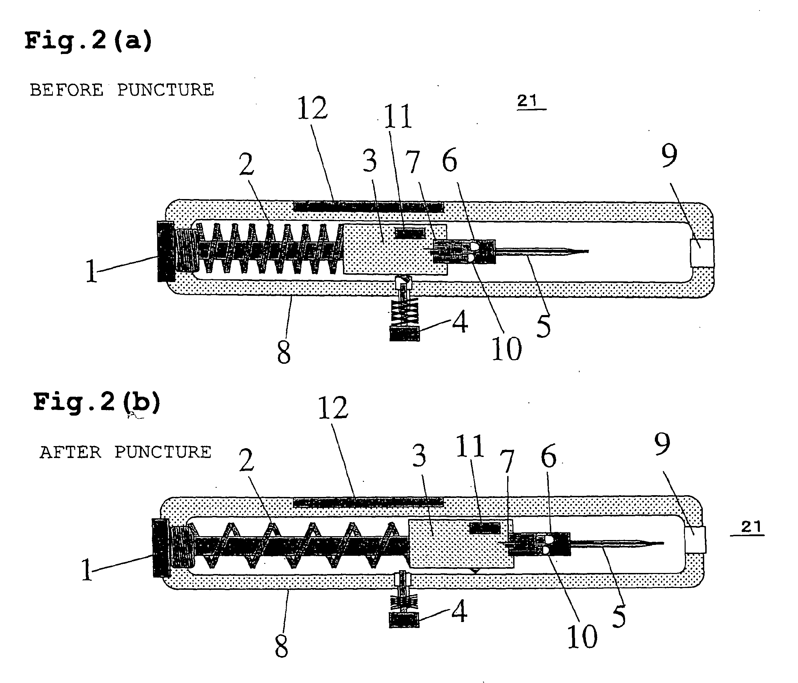

Figure 1 is a diagram showing the construction of a living body information measuring device 21 in the first embodiment of the present invention. Figures 2(a) and 2(b) show cross-sectional views of the livingbody information measuring device 21. Figure 2 (a) is a cross-sectional view of the living body information measuring device 21 before puncture, and Figure 2(b) is a cross-sectional view of the living body information measuring device 21 after puncture. For ease of understanding, an enlarged view of a portion in the vicinity of an opening 9 of a lancet main body 8 in particular is added to Figure 1.

As shown in Figures 2 (a) and 2 (b) , a spring 2, a puncturing needle holder 3, a light source 6, a battery 7, a detector 10, an computation unit 11 and a display 12 are incorporated in the lancet main body 8, which is a casing of the living body information measuring device 21.

The opening 9 through which the puncturing needle 5 moves in and out of the lancet main body 8 is provided at the extreme tip of the lancet main body 8. A spring compressing button 1 is provided at the end of the lancet main body 8 opposite from the opening 9. The spring compressing button 1 is connected to the spring 2 so as to be capable of compressing the spring 2. A trigger button 4 is placed on a side surface of the lancet main body 8. The puncturing needle 5 is detachably attached to the puncturing needle holder 3. The puncturing needle 5 has a hollowed inner portion as represented by a hollow 13 shown in Figure 1 , so that light can pass through the inner portion of the puncturing needle 5.

The inside diameter of the puncturing needle 5 may be set to any value. However, it is preferred that the inside diameter be equal to or smaller than 0. 5 mm. It is preferable to minimize the distance between the light source or the detector and the needle extreme end. Preferably, the distance therebetween is equal to or smaller than 10 mm.

The computation unit 11 is incorporated in the puncturing needle holder 3. The light source 6, the battery 7 and the detector 10 are attached to the puncturing needle holder 3.

The battery 7, the light source 6, the detector 10, the computation unit 11 and the display 12 are connected to one another by power supply wiring, which is not illustrated. The detector 10 and the computation unit 11 are connected to each other by signal wiring for signal exchange. The computation unit 11 and the display 12 are connected to each other by signal wiring for signal exchange.

A laser diode, an LED or the like may be used as the light source 6. A photoelectric conversion element, e.g., a photodiode or a CdS, or the like may be used as the detector 10.

The spring compressing button 1, the spring 2, the puncturing needle holder 3 and the trigger button 4 in this embodiment are an example of the lancet drive mechanism of the present invention; the lancet main body 8 in this embodiment is an example of the casing of the present invention; the detector 10 in this embodiment is an example of the photodetector of the present invention; and the puncturing needle 5 in this embodiment is an example of the lancet needle of the present invention.

The operation of this embodiment will now be described.

Description will be first made of the operation when the living body information measuring device 21 punctures a part of the body such as an inner surface portion of a finger or an earlobe from which a blood sample is to be obtained.

A needle of 28G (gauge) is used as the puncturing needle 5 . A finger tip is first lanced by using the puncturing needle 5. That is, it is assumed that the living body information measuring device 21 is initially in the state shown in Figure 2 (b) . However, the puncturing needle 5 is not yet attached to the puncturing needle holder 3. The spring 2 is in an extended state. In this state, the puncturing needle 5 is attached to the puncturing needle holder 3. The living body information measuring device 21 is thereby set in the state shown in Figure 2(b).

Subsequently, the lancet main body 8 is held and the spring compressing button 1 is pulled in a direction away from the lancet main body 8. With the movement of the spring compressing button 1 in the direction away from the lancet main body 8, the spring 2 is compressed. The spring compressing button 1 is pulled until a projection provided on the puncturing needle holder 3 moves beyond (the position of) the trigger button 4 in a direction toward the spring compressing button 1. The projection provided on the puncturing needle holder 3 is then caught by the trigger button 4, thereby fixing the spring 2 in a compressed state. After pulling the spring compressing button 1 until the projection provided on the puncturing needle holder 3 moves beyond the trigger button 4 in a direction toward the spring compressing button 1, the spring compressing button 1 is returned to the position at which it is in close contact with the lancet main body 8. The state shown in Figure 2 (a) is thus attained.

Next, the living body information measuring device 21 is moved to the part of the body, e. g. , an inner surface portion of a finger or an earlobe, the opening 9 provided in the lancet main body 8 is positioned on the part of the body from which a blood sample is to be obtained, and the trigger button 4 is thereafter pressed. Then, the projection provided on the puncturing needle holder 3 is then released from the trigger button 4, the spring 2 extends, and the puncturing needle 5 moves out of the opening to puncture the part of the body from which a blood sample is to be obtained. Thus, the living body information measuring device 21 is set in the state shown in Figure 2 (b) .

When the part of the body from which a blood sample is to be obtained is punctured with the puncturing needle 5, substantially no part of blood bleeding from the part of the body from which a blood sample is to be obtained is attached to the puncturing needle 5. Therefore, the inside of the puncturing needle 5 is still in the hollow state even after puncture.

The operation when the living body information measuring device 21 punctures a part of the body such as an inner surface portion of a finger or an earlobe from which a blood sample is to be obtained has been described.

Description will next be made of the operation at the time of measurement of the blood sugar value.

That is, blood bleeding from a part of the body from which a blood sample is obtained is applied dropwise to a reaction portion of an optical blood sugar sensor 20. The blood applied dropwise to a reaction portion of an optical blood sugar sensor 20 reacts with a reaction reagent through a blood corpuscle filter to exhibit a change in coloration.

When a blood sugar value is measured, the living body information measuring device 21 is first moved to the reaction portion of the optical blood sugar sensor 20 and is positioned on the same, as shown in Figure 1. At this time, the living body information measuring device 21 is in the state shown in Figure 2 (b) .

Subsequently, a measurement button (not shown) provided on the living body information measuring device 21 is pressed to emit light from the light source 6. The emitted light passes through the hollowed inner portion of the puncturing needle 5 and is emitted from the opening 9 to irradiate the reaction portion of the optical blood sugar sensor 20. The light radiated to the reaction portion enters the opening 9 after reflection and absorption by the reaction portion, passes through the hollowed inner portion of the puncturing needle 5 and reaches the detector 10. A signal detected by the detector 10 is sent to the computation unit 11. That is, the light source 6 irradiates the optical blood sugar sensor 20 in a cycle of 60 Hz through the puncturing needle 5 with, and is simultaneouslymeasured at 60 Hz in a different phase through the same puncturing needle 5.

The computation unit 11 obtains the glucose concentration 11 from a signal representing optical information such as the quantity of light sent from the detector 10, by using a correspondence table which is held in a memory held in the computation unit 11 and in which optical information such as the quantity of light and the glucose concentration are associated with each other. Information on the glucose concentration obtained by the computation unit 11 is sent to the display 12.

The display 12 displays the glucose concentration sent from the computation unit 11.

In some cases, a small amount of blood applied dropwise to the reaction portion of the optical blood sugar sensor 20 does not spread over the entire dropwise-application portion, and a change in coloration is not caused at some areas in the reaction portion of the optical blood sugar sensor 20.

In such a case, in the conventional optical blood sugar meter, measurement noise is generated if light emitted from the optical blood sugar meter is radiated to an area on the reaction portion of an optical blood sugar sensor at which no change in coloration is caused, because measurement is performed by attaching the optical blood sugar sensor to the optical blood sugar meter.

However, the optical blood sugar sensor is not attached to the living body information measuring device 21 in this embodiment. Therefore, adjustment is easily made by moving the living information measuring device 21 so that light which has passed through the inner portion of the puncturing needle 5 is radiated to the reaction portion of the optical blood sugar sensor 20 at an area where the coloring reaction has occurred, even if the light which has passed through the interior of the puncturing needle 5 has been radiated to the reaction portion at an area where the coloration reaction has not occurred. That is, the spot of light radiated to the reaction portion of the optical blood sugar sensor 20 can be visually checked and can therefore be moved, by moving living body information measuring device 21, an area at which the coloration reaction has occurred. Also, since light is radiated to the optical blood sugar sensor 20 from the extreme end of the puncturing needle 5, the puncturing needle 5 can be used as a mark for positioning of the spot of light to an area on the reaction portion of the optical blood sugar sensor 20 at which the coloration reaction has occurred. Aftermoving the spot of light to an area at which the coloration reaction has occurred, the measurement button is again pressed; whereby measurement of optical information at an area at which the coloration reaction has not occurred can be avoided. Therefore, no measurement noise is generated. Thus, the living body information measuring device 21 of this embodiment can be freely moved relative to the optical blood sugar sensor 20 to enable optical information at an area at which the coloration reaction has occurred to be easily measured.

This embodiment has been described by assuming that the puncturing needle 5 is hollow. However, the puncturing needle 5 is not limited to this, and it may have a groove.

Thus, in the living body information measuring device 21 of this embodiment, the light source irradiates the optical blood sugar sensor 20 through the hollow or the groove in the puncturing needle 5 having such a shape as to be capable of puncture, and having the groove or the hollow, and reflected light or fluorescent light thereby caused can be guided to the detector 10 through the hollow or the groove in the puncturing needle 5.

The living body information measuring device 21 of this embodiment has such a structure that the lancet device and the measuring device are combined into one and, therefore, has improved portability. Also, irradiation light can be made to strike only the reaction portion on the optical blood sugar sensor 20 where the reaction between blood and the reagent occurs, thus preventing noise from being generated from a non-reaction portion.

This embodiment has been described with respect to measurement of the blood sugar value. However, the present invention is not limited to this. Any living body information other than the blood sugar value may be measured.

This embodiment has been described by assuming that light is emitted from the light source 6 when the measurement button is pressed once, and that detection of optical information such as the quantity of light is performed with the detector 10 when the measurement button is again pressed. However, the present invention is not limited to this. When the measurement button is pressed once, light is emitted from the light source 6, detection of optical information such as the quantityof light is simultaneously performed with the detector 10, living body information such as glucose concentration is obtained by the computation unit 11, and living body information such as the glucose concentration is displayed on the display 12. This operation is repeated for a predetermined time period. After a lapse of the predetermined time period, the display 12 holds the maximum value of obtained living body information, e.g., glucose concentration. By doing so, even if the living body information measuring device 21 is freely moved relative to the optical blood sugar sensor 20, it is possible to know the value of the living body information, e.g., glucose concentration obtained when light is radiated from the puncturing needle 5 to the reaction portion of the optical blood sugar sensor 20 at an area where coloration reaction has occurred to a sufficiently high degree, since the maximum value of living body information, e.g., the obtained glucose concentration is held and the held maximum value is displayed on the display 12.

This embodiment has been described by assuming that the computation unit 11 and the display 12 are incorporated in the lancet main body 8 which is the casing of the living body information measuring device 21. However, the present invention is not limited to this. An arrangementmaybe adopted in which the computation unit 11 and the display 12 are not incorporated in the lancet main body 8, which is the casing of the living body information measuring device 21. In such a case, the computation unit 11 and the display 12 are provided in an external unit such as a personal computer. Also, the living body information measuring device 21 sends to an external unit such as a personal computer a signal representing optical information such as the quantity of light sent from the detector 10, and the external unit, which is a personal computer or the like, has functions corresponding to those of the computation unit 11 and the display 12. Also, in such a case, the living body information measuring device 21 has a communication interface for sending, to the external unit, which is a personal computer or the like, the signal representing optical information, e.g. , the quantity of light sent from the detector 10. The communication interface may be one for performing wireless communication (e.g.,Bluetooth, infrared communication or the like) with the external unit, which is a personal computer or the like, or one for performing wired communication (e.g. , USB or the like) with the external unit, which is a personal computer or the like.

Thus, even in a case where the living body information measuring device 21 is arranged without the computation unit 11 and the display 12, a signal representing optical information such as the quantity of light sent from the detector 10 can be sent to an external unit, living body information such as the glucose concentration can be obtained in the external unit from the signal representing the optical information, e. g. , the quantity of light sent from the detector 10 by using a correspondence table which is held in a memory in the external unit and in which optical information such as the quantity of light and the glucose concentration are associated with each other, and information on the obtained living body information, e.g., the glucose concentration can be displayed in the external unit. Thus, even in the case of an arrangement in which the living body information measuring device 21 is not provided with the computation unit 11 and the display 12, the same effects as those of the first embodiment can be obtained.

Further, this embodiment has been described by assuming that the puncturing needle 5 is detachably attached to the puncturing needle holder 3, but the present invention is not limited to this. An arrangement may be adopted in which the puncturing needle 5 is kept in the state of being attached to the puncturing needle holder 3 and cannot be pulled out of the puncturing needle holder 3.

Further, this embodiment has been described by assuming that an opening is provided at the extreme tip of the lancet main body 8, but the present invention is not limited to this. For example, the lancet main body 8 may have a cubic shape. In a case where the lancet main body 8 has a cubic shape, the opening 9 may be provided in a central portion of one of the six faces of the cubic shape of the lancet main body 8. The lancet main body 8 may have a spherical shape. In a case where the lancet main body 8 has a spherical shape, the opening 9 may be provided in the spherical surface of the lancet main body 8. Thus, even in a case where the opening 9 is provided in a portion other than a portion of the lancet main body 8 at the extreme tip, the same effects as those in this embodiment can be obtained.

Further, the lancet mechanism of the present invention is not limited to the spring compressing button 1, the spring 2, the puncturing needle holder 3 and the trigger button 4. A pneumatic cylinder may be used instead of the spring 2 and a piston capable of being inserted in the pneumatic cylinder to compress air in the pneumatic cylinder may be used instead of the spring compressing button 1. In this case, air is introduced into the cylinder by pulling the piston, and the air introduced into the cylinder is compressed by pushing the piston. In a state where the air introduced into the cylinder is compressed, the trigger button 4 is pressed to cause the puncturing needle holder 3 to move toward the opening 6 by the repulsive force of the compressed air. Thus, the lancet drive mechanism of the present invention may be constructed so as to compress air and drive the puncturing needle 5 by the compressed air.

### (Second Embodiment)

Description will next be made of the second embodiment.

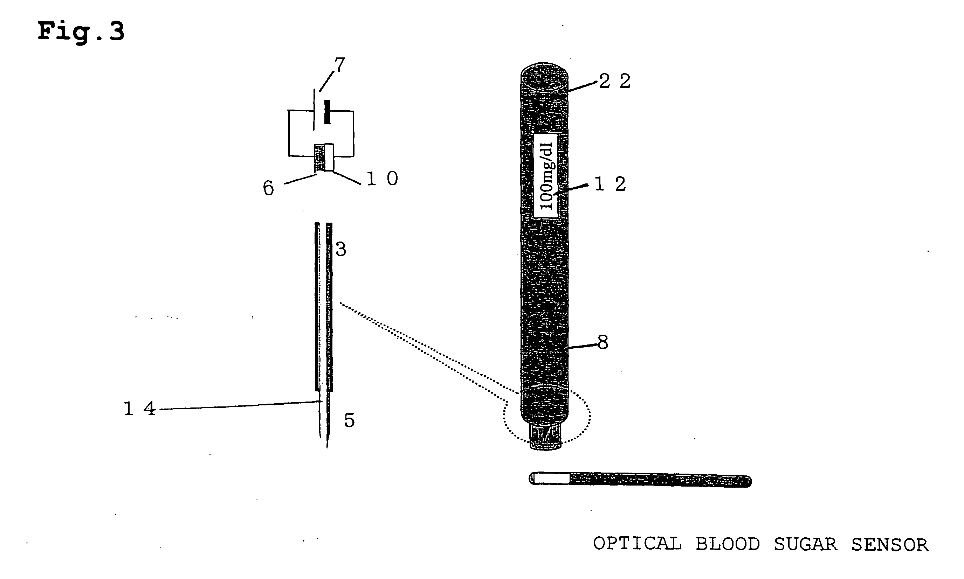

Figure 3 shows the construction of a living body information measuring device 22 of the second embodiment.

The living body information measuring device 22 of the second embodiment differs from the first embodiment in that an optical fiber as represented by an optical fiber 14 shown in Figure 3 is inserted in the puncturing needle 5 of the living body information measuring device 22 of the second embodiment.

The living body information measuring device 22 as shown in section is the same as the first embodiment shown in Figure 2 except that the optical fiber 14 is inserted in the puncturing needle 5.

The operation of this embodiment will be described mainly with respect to points of difference from the first embodiment.

The operation of the living body information measuring device 22 of the second embodiment is the same as that of the first embodiment except for points described below.

That is, at the time of measurement of the blood sugar value, the living body information measuring device 22 of the second embodiment can guide light emitted from the light source 6 to the reaction portion of the optical blood sugar sensor 20 with higher efficiency, because the optical fiber 14 is inserted in the puncturing needle 5. Also, light reflected from the reaction portion of the optical blood sugar sensor 20 can be guided to the detector 10 with higher efficiency, because the optical fiber 14 is inserted in the puncturing needle 5. Thus, the living body information measuring device 22 is capable of measuring the blood sugar value with improved accuracy in comparison with the living body information measuring device 21 of the first embodiment.

This embodiment has been described by assuming that the optical fiber 14 is inserted in the puncturing needle 5. However, the present invention is not limited to this. A groove may be provided in the puncturing needle 5 and the optical fiber 14 may be inserted in this groove.

Thus, a photoconductive member such as an optical fiber is fitted or inserted in the hollow or the groove in the puncturing needle 5, the light source irradiates the optical blood sugar sensor 20 through the puncturing needle 5, and reflected light or fluorescent light thereby produced can be guided to the detector 10 through the puncturing needle 5 with higher efficiency.

The second embodiment has been described by assuming that the puncturing needle 5 is used in the living body information measuring device 22. However, the present invention is not limited to this. The puncturing needle 5 may be used for a different purpose.

### (Third Embodiment)

Description will next be made of the third embodiment.

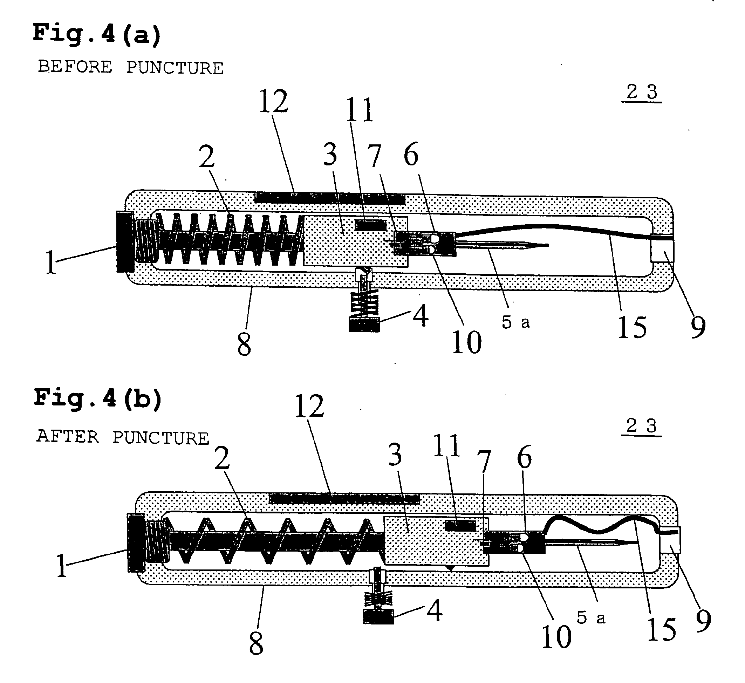

Figure 4 shows the cross-section of a living body information measuring device 23 of the third embodiment.

The living body information measuring device 23 of the third embodiment differs from the living body information measuring device 21 of the first embodiment in that a puncturing needle 5a in the third embodiment is not hollow but an optical fiber 15 is led from the light source 6 and the detector 10 to the opening 9 without being passed through the inner portion of the puncturing needle 5a. The construction of the living body information measuring device 23 of the third embodiment is the same as that described above with respect to the embodiment with reference to Figure 1 except that the optical fiber 15 is provided.

In other respects, this embodiment is the same as the first embodiment. Description of the same details will not be repeated.

The puncturing needle 5a of this embodiment is an example of the lancet needle of the present invention.

The operation of this embodiment will next be described.

The operation of the living body in formation measuring device 23 of the third embodiment is the same as that of the first embodiment except for points described below.

That is, when the living body information measuring device 23 of the third embodiment measures the blood sugar value, it guides light emitted from the light source 6 to the opening 9 by the optical fiber 15 provided separately from the puncturing needle 5a instead of causing it to pass through an inner portion of the puncturing needle 5a. The light guided to the opening 9 is radiated to the reaction portion of the optical blood sugar sensor 20.

After the light reflected from the reaction portion of the optical blood sugar sensor 20 reaches the opening 9, it is guided by the optical fiber 15 to the detector 10.

Thus, light issuing from the light source 6 is guided by the optical fiber 15 instead of being guided by the puncturing needle 5a and light reflected from the reaction portion of the optical blood sugar sensor 20 is guided to the detector 10 by the optical fiber 15. Also in this case, the same effects as those in the first embodiment or the second embodiment can be obtained.

### Industrial Applicability

As is apparent from the foregoing, the present invention can provide a living body information measuring device and a living body information measuring method both having improved portability.

The present invention can also provide a living body information measuring device, a needle and a living body information measuring method enabling accurate measurement even when a small amount of blood is applied dropwise.

## Claims

1. A living body information measuring device comprising:
a casing;
a light source which is accommodated in said casing and which emits light;
a photodetector which is accommodated in said casing and which detects light; and
a lancet drive mechanism which is accommodated in said casing and which drives a lancet needle,
wherein said lancet needle moves in and out an opening provided in said casing;
the light emitted from said light source is emitted from said opening; and
light entering said opening reaches said detector.

2. The living body information measuring device according to claim 1, wherein the light emitted from said opening is reflected and absorbed by a reagent portion of a sensor; and
the light entering said opening is light reflected by the reagent portion of said sensor.

3. The living body information measuring device according to claim 1, wherein an inner portion of the lancet needle is capable of transmitting light;
the light emitted from said light source passes through the inner portion of said lancet needle and is emitted from said opening; and
the light entering said opening reaches said photodetector by passing through the inner portion of said lancet needle.

4. The living body information measuring device according to claim 3, wherein an optical fiber which guides light is inserted in the inner portion of said lancet needle.

5. The living body information measuring device according to claim 1, wherein the light emitted from said light source is guided to said opening by an optical fiber and is emitted from said opening; and
the light entering said opening is guided to the photodetector by the optical fiber.

6. The living body information measuring device according to claim 1, comprising a computation unit which is accommodated in said casing and which figures out living body information from a result detected by said detector; and
a display which is accommodated in said casing and which displays the figured-out living body information.

7. A living body information measuring method using a living body information measuring device having:
a casing;
a light source which is accommodated in said casing and which emits light;
a photodetector which is accommodated in said casing and which detects light; and
a lancet drive mechanism which is accommodated in said casing and which drives a lancet needle, said method comprising:
a step of causing said lancet needle to move in and out an opening provided in said casing;
a step of causing the light emitted from said light source to be emitted from said opening; and
a step of causing light entering said opening to reach said detector.

## Amended claims

### Amended claims under Art. 19.1 PCT

1. Amended) A living body information measuring device comprising:
a casing;
a light source which is accommodated in said casing and which emits light;
a photodetector which is accommodated in said casing and which detects light; and
a lancet drive mechanism which is accommodated in said casing and which drives a lancet needle,
wherein said lancet needle moves in and out an opening provided in said casing;
the light emitted from said light source is emitted from said opening;
light entering said opening reaches said detector;
the light emitted from said opening is reflected and absorbed by a reagent portion of a sensor; and
the light entering said opening is light reflected by the reagent portion of said sensor.

2. (Deleted)

3. The living body information measuring device according to claim 1, wherein an inner portion of the lancet needle is capable of transmitting light;
the light emitted from said light source passes through the inner portion of said lancet needle and is emitted from said opening; and
the light entering said opening reaches said photodetector by passing through the inner portion of said lancet needle.

4. The living body information measuring device according to claim 3, wherein an optical fiber which guides light is inserted in the inner portion of said lancet needle.

5. The living body information measuring device according to claim 1 , wherein the light emitted from said light source is guided to said opening by an optical fiber and is emitted from said opening; and
the light entering said opening is guided to the photodetector by the optical fiber.

6. The living body information measuring device according to claim 1, comprising a computation unit which is accommodated in said casing and which figures out living body information from a result detected by said detector; and
a display which is accommodated in said casing and which displays the figured-out living body information.

7. (Amended) A living body information measuring method using a living body information measuring device having:
a casing;
a light source which is accommodated in said casing and which emits light;
a photodetector which is accommodated in said casing and which detects light; and
a lancet drive mechanism which is accommodated in said casing and which drives a lancet needle, said method comprising:
a step of causing said lancet needle to move in and out an opening provided in said casing;
a step of causing the light emitted from said light source to be emitted from said opening;
a step of causing light entering said opening to reach said detector,
wherein the light emitted from said opening is reflected and absorbed by a reagent portion of a sensor; and
the light entering said opening is light reflected by the reagent portion of said sensor.
